(19) Europäisches Patentamt
European Patent Office
Office européen des brevets

(11) Veröffentlichungsnummer: **0 114 608**
**A2**

(12) # EUROPÄISCHE PATENTANMELDUNG

(21) Anmeldenummer: 84100177.9

(22) Anmeldetag: 10.01.84

(51) Int. Cl.³: C 07 D 249/08
A 01 N 43/64

(30) Priorität: 22.01.83 DE 3302120

(43) Veröffentlichungstag der Anmeldung: 01.08.84 Patentblatt 84/31

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kraatz, Udo, Dr.
Andreasstrasse 22a
D-5090 Leverkusen 1(DE)

(72) Erfinder: Reiser, Wolf, Dr.
Kiebitzweg 12a
D-5600 Wuppertal 1(DE)

(72) Erfinder: Büchel, Karl Heinz, Prof. Dr.
Dabringhausener Strasse 42
D-5093 Burscheid(DE)

(72) Erfinder: Brandes, Wilhelm, Dr.
Eichendorffstrasse 3
D-5653 Leichlingen 1(DE)

(72) Erfinder: Reinecke, Paul, Dr.
Lessingstrasse 11
D-5090 Leverkusen 3(DE)

(54) **(+)-Antipode des (E)-1-Cyclohexyl-4,4-dimethyl-3-hydroxy-2-(1,2,4-triazol-1-yl)-pent-1-ens.**

(57) Der neue (+)-Antipode des (E)-1-Cyclohexyl-4,4-dimethyl-3-hydroxy-2-(1,2,4-triazol-1-yl)-pent-1-ens der Formel

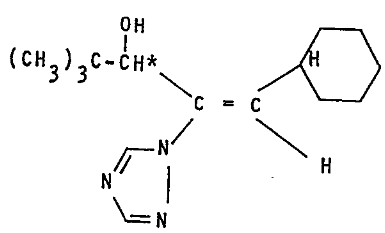

(I)

besitzt sehr gute fungizide Eigenschaften. Der optisch aktive Wirkstoff der Formel (I) läßt sich aus dem racemischen (E)-1-Cyclohexyl-4,4-dimethyl-3-hydroxy-2-(1,2,4-triazol-1-yl)-pent-1-en durch Racematspaltung herstellen.

EP 0 114 608 A2

0114608

BAYER AKTIENGESELLSCHAFT 5090 Leverkusen, Bayerwerk

Konzernverwaltung RP
Patentabteilung

Dü-klu/c
Ia

# (+)-Antipode des (E)-1-Cyclohexyl-4,4-dimethyl-3-hydroxy-2-(1,2,4-triazol-1-yl)-pent-1-ens

Die vorliegende Erfindung betrifft den neuen (+)-Antipoden*) des (E)-1-Cyclohexyl-4,4-dimethyl-3-hydroxy-2-(1,2,4-triazol-1-yl)-pent-1-ens, ein Verfahren zu dessen Herstellung und dessen Verwendung als Fungizid.

Es ist bereits bekannt, daß das Racemat des 1-Cyclohexyl-4,4-dimethyl-3-hydroxy-2-(1,2,4-triazol-1-yl)-pent-1-ens fungizide Eigenschaften besitzt (vgl. DE-OS 29 06 061). Die Wirksamkeit dieses Produktes ist gut, jedoch ist der erzielte Effekt bei sehr niedrigen Aufwandmengen nicht immer befriedigend.

---

*) Unter dem (+)-Antipoden ist hier jeweils dasjenige Enantiomere zu verstehen, das die Schwingungsebene von linear polarisiertem Licht der Natrium-D-Linie nach rechts dreht.

Weiterhin ist bekannt, daß 1-(Cyclohex-3-en-1-yliden)-4,4-dimethyl-3-hydroxy-2-(1,2,4-triazol-1-yl)-pentan zur bekämpfung von Pilzen eingesetzt werden kann (vgl. DE-OS 29 05 981). Bei niedrigen Aufwandmengen ist allerdings auch die Wirksamkeit dieses Stoffes nicht immer ausreichend.

Es wurde nun der (+)-Antipode des (E)-1-Cyclohexyl-4,4-dimethyl-3-hydroxy-2-(1,2,4-triazol-1-yl)-pent-1-ens der Formel

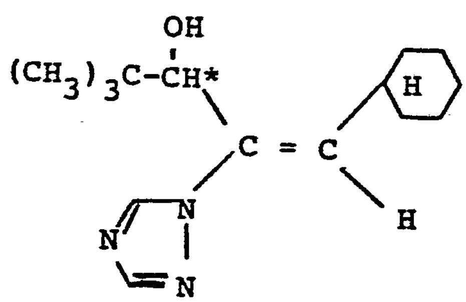

(I)

gefunden.

Weiterhin wurde gefunden, daß man den neuen (+)-Antipoden des (E)-1-Cyclohexyl-4,4-dimethyl-3-hydroxy-2-(1,2,4-triazol-1-yl)-pent-1-ens der Formel (I) erhält, wenn man in einer ersten Stufe racemisches (E)-1-Cyclohexyl-4,4-dimethyl-3-hydroxy-2-(1,2,4-triazol-1-yl)-pent-1-en der Formel

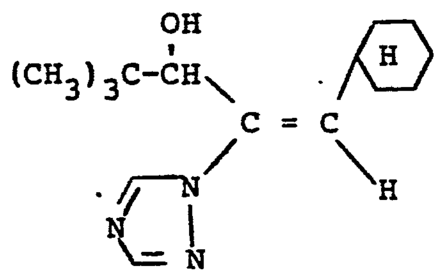

(Ia)

mit einem optisch aktiven Säurehalogenid der Formel

$$R - X - Hal \qquad (II)$$

in welcher

R für einen optisch aktiven Rest steht,
X für $-CO-$, $-SO_2-$ oder $-O-CH_2-CO-$ steht und
Hal für Halogen steht,

in Gegenwart eines Verdünnungsmittels und gegebenenfalls in Gegenwart von Basen umsetzt, dann die so erhaltenen diastereomeren Ester der Formel

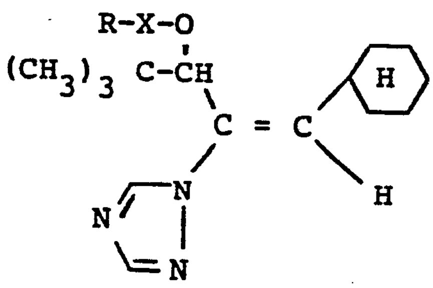

(III)

(Diastereomeren-Gemisch)

in welcher

R und X die oben angegebene Bedeutung haben,

aufgrund ihrer unterschiedlichen physikalischen Eigenschaften trennt

und danach in einer zweiten Stufe den (+)-Antipoden des (E)-1-Cyclohexyl-4,4-dimethyl-3-hydroxy-2-(1,2,4-triazol-1-yl)-pent-1-ens aus dem entsprechenden Ester mit Hilfe von Basen in Gegenwart eines Verdünnungsmittels in Freiheit setzt.

Schließlich wurde gefunden, daß sich der neue (+)-Antipode des (E)-1-Cyclohexyl-4,4-dimethyl-3-hydroxy-2-(1,2,4-triazol-1-yl)-pent-1-ens der Formel (I) durch eine hervorragende fungizide Wirksamkeit auszeichnet.

Überraschenderweise besitzt der neue (+)-Antipode des (E)-1-Cyclohexyl-4,4-dimethyl-3-hydroxy-2-(1,2,4-triazol-1-yl)-pent-1-ens der Formel (I) bessere fungizide Eigenschaften als das entsprechende Racemat und das 1-(Cyclohex-3-en-1-yliden)-4,4-dimethyl-3-hydroxy-2-(1,2,4-triazol-1-yl)-pentan, welche Verbindungen aus dem Stand der Technik als Fungizide bekannt sind. Im übrigen konnte nicht erwartet werden, daß der erfindungsgemäße Wirkstoff sich durch sehr gute fungizide Wirksamkeit hervorhebt, während der (-)-Antipode des (E)-1-Cyclohexyl-4,4-dimethyl-3-hydroxy-2-(1,2,4-triazol-1-yl)-pent-1-ens als Fungizid deutlich weniger wirksam ist.

Die erfindungsgemäße Verbindung ist durch die Formel (I) definiert. In dieser Formel ist das asymmetrische Kohlenstoffatom durch ein (*) gekennzeichnet.

Verwendet man das racemische (E)-1-Cyclohexyl-4,4-Dimethyl-3-hydroxy-2-(1,2,4-triazol-1-yl)-pent-1-en als Ausgangsstoff, (-)-Menth-3-yloxy-acetylchlorid als optisch aktives Säurehalogenid, Triethylamin und 4-Dimethylaminopyridin (DMAP) als Hilfsbasen zur Veresterung (1. Stufe) und ein Gemisch aus Natriumhydroxid, Wasser und Methanol zur Esterverseifung (2. Stufe), so kann der Reaktionsablauf des erfindungsgemäßen Verfahrens durch das folgende Formelschema wiedergegeben werden:

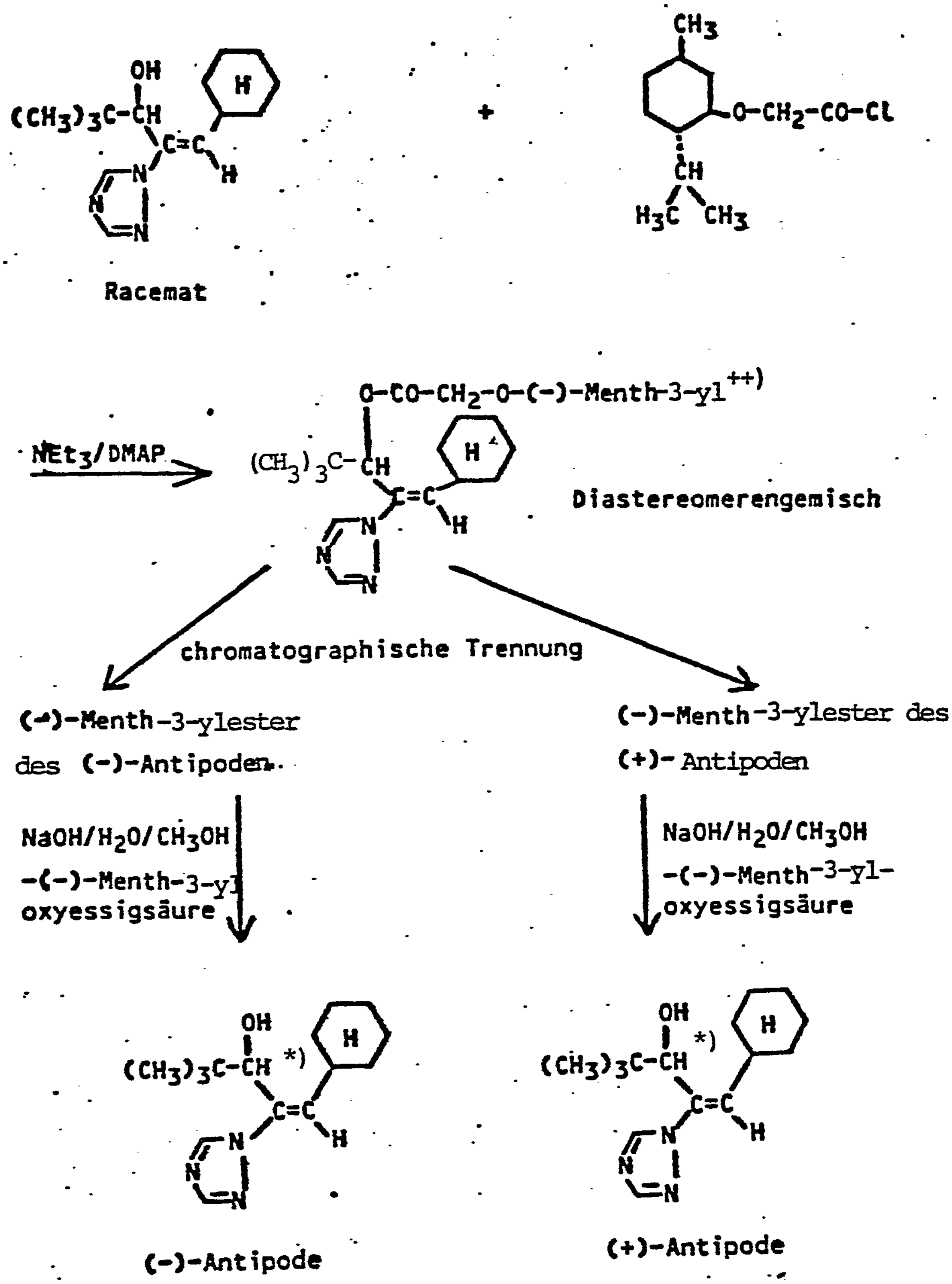
Racemat
+
NEt3/DMAP
Diastereomerengemisch
chromatographische Trennung
(-)-Menth-3-ylester des (-)-Antipoden
(-)-Menth-3-ylester des (+)-Antipoden
NaOH/H2O/CH3OH
-(-)-Menth-3-yl-oxyessigsäure
NaOH/H2O/CH3OH
-(-)-Menth-3-yl-oxyessigsäure
(-)-Antipode
(+)-Antipode

---

++) (-)-Menth = 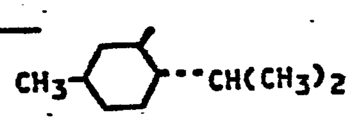

Das bei der Durchführung des erfindungsgemäßen Verfahrens als Ausgangsstoff benötigte Racemat des (E)-1-Cyclohexyl-4,4-dimethyl-3-hydroxy-2-(1,2,4-triazol-1-yl)-pent-1-ens der Formel (Ia) ist bekannt (vgl. DE-OS 29 06 061).

Die bei dem erfindungsgemäßen Verfahren weiterhin als Ausgangssubstanzen benötigten optisch aktiven Säurehalogenide sind durch die Formel (II) allgemein definiert. In dieser Formel steht R vorzugsweise für (+)-3-Brom-campher-8-yl, (+)-Campher-10-yl oder (-)-Menth-3-yl. X steht für die Reste -CO-, $-SO_2-$ und $-O-CH_2-CO-$, und Hal steht vorzugsweise für Chlor oder Brom.

Als Beispiele für Verbindungen der Formel (II) seien genannt:

(+)-3-Brom-campher-8-sulfonsäurechlorid,
(+)-Campher-10-sulfonsäurechlorid
(+)-3-Brom-campher-8-sulfonsäurebromid
(+)-Campher-10-sulfonsäurebromid
(-)-Menth-3-yl-oxyacetylchlorid
(-)-Menth-3-yl-oxyacetylbromid.

Die optisch aktiven Säurehalogenide sind bekannt oder lassen sich nach bekannten Methoden in einfacher Weise herstellen.

Als Verdünnungsmittel kommen für die Durchführung der 1. Stufe (Veresterung) des erfindungsgemäßen Verfahrens alle inerten organischen Lösungsmittel in Frage. Hierzu gehören vorzugsweise Kohlenwasserstoffe, wie Benzin,

Benzol, Toluol oder Xylol, halogenierte Kohlenwasserstoffe, wie Dichlormethan, Chloroform, Tetrachlorkohlenstoff, Chlorbenzol oder Dichlorbenzol, Ether, wie Diethylether, Tetrahydrofuran oder Dioxan, Nitrile, wie Acetonitril oder Propionitril, oder Ester, wie Essigsäureethylester.

Die erste Stufe des erfindungsgemäßen Verfahrens wird vorzugsweise in Gegenwart von Basen durchgeführt. Hierbei können alle üblichen organischen oder anorganischen Basen eingesetzt werden. Vorzugsweise verwendbar sind Alkalihydroxide oder Alkalicarbonate, wie beispielsweise Natriumhydroxid, Natriumcarbonat oder Natriumhydrogencarbonat, ferner niedere tertiäre Alkylamine, Cycloalkylamine, Arylalkylamine oder Arylamine, wie beispielsweise Triethylamin, N,N-Dimethylbenzylamin, Pyridin, 1,4-Diazabicyclo-[2,2,2]-octan oder 1,5-Diazabicyclo-[4,3,0]-non-5-en. Besonders bevorzugt verwendet man ein Gemisch aus Triethylamin und dem hochnucleophilen 4-Dimethylaminopyridin.

Die Reaktionstemperaturen können bei der Durchführung der 1. Stufe des erfindungsgemäßen Verfahrens in einem größeren Bereich variiert werden. Im allgemeinen arbeitet man zwischen -20°C und +120°C, vorzugsweise zwischen 0°C und 60°C.

Bei der Durchführung der 1. Stufe des erfindungsgemäßen Verfahrens setzt man auf 1 Mol der racemischen Ausgangsverbindung der Formel (Ia) vorzügsweise 1 bis 1,5 Mol

optisch aktives Säurehalogenid der Formel (II) und 2 bis 3 Mol Base ein. Die Isolierung des Diastereomeren-Gemisches erfolgt nach üblichen Methoden. Im allgemeinen geht man so vor, daß man nach beendeter Umsetzung mit Wasser versetzt, das entstehende Gemisch mehrfach mit einem mit Wasser wenig mischbaren organischen Lösungsmittel extrahiert, die vereinigten organischen Phasen trocknet und unter vermindertem Druck einengt.

Die Trennung der diastereomeren Ester der Formel (III) kann nach den für derartige Zwecke geeigneten Methoden vorgenommen werden, also zum Beispiel durch fraktionierte Kristallisation oder auch mit Hilfe von chromatographischen Verfahren.

Besonders bevorzugt wendet man säurenchromatrographische Trennverfahren an, wie z.B. die Hochdruckfiltration über eine Kieselgelsäure mit einem Elutionsgemisch aus Hexan, Tetrachlorkohlenstoff und Propionitril.

Als Verdünnungsmittel für die 2. Stufe (Esterverseifung) des erfindungsgemäßen Verfahrens kommen ebenfalls inerte organische Lösungsmittel in Frage. Besonders bevorzugt verwendet man Alkohole, wie beispielsweise Methanol, Ethanol oder Propanol.

Die Freisetzung des erfindungsgemäßen Wirkstoffes erfolgt in der zweiten Stufe des erfindungsgemäßen Verfahrens mit Hilfe von Basen. Bevorzugt verwendet man dabei starke wäßrige anorganische Basen, wie Natriumhydroxid oder Kaliumhydroxid in Wasser.

Die Reaktionstemperaturen können bei der Durchführung der 2. Stufe des erfindungsgemäßen Verfahrens ebenfalls in einem größeren Bereich variiert werden. Im allgemeinen arbeitet man zwischen 0°C und 80°C, vorzugsweise zwischen 10°C und 60°C.

Bei der Durchführung der 2. Stufe des erfindungsgemäßen Verfahrens setzt man auf 1 Mol des jeweiligen diastereomeren Esters der Formel (III) im allgemeinen 1 bis 3 Mol, vorzugsweise 1 bis 2 Mol Base ein.

Die Isolierung des erfindungsgemäßen Wirkstoffes erfolgt nach üblichen Methoden. Im allgemeinen verfährt man in der Weise, daß man das Reaktionsgemisch mit Wasser versetzt, dann mehrfach mit einem mit Wasser wenig mischbaren organischen Lösungsmittel extrahiert, die vereinigten organischen Phasen trocknet und durch Abziehen des Lösungsmittels unter vermindertem Druck einengt und den verbleibenden Rückstand gegebenenfalls durch Umkristallisation oder durch Waschen mit einem organischen Lösungsmittel von eventuell vorhandenen Verunreinigungen befreit.

Bei der Durchführung der zweiten Stufe des erfindungsgemäßen Verfahrens wird jeweils derjenige diastereomere Ester der Formel (III) eingesetzt, aus dem durch Behandlung mit Base der erfindungsgemäße (+)-Antipode des (E)-1-Cyclohexyl-4,4-dimethyl-3-hydroxy-2-(1,2,4-triazol-1-yl)-pent-1-ens in Freiheit gesetzt wird.

Nach dem erfindungsgemäßen Verfahren läßt sich auch der (-)-Antipode des (E)-1-Cyclohexyl-3-hydroxy-2-(1,2,4-triazol-1-yl)-pent-1-ens darstellen. Man geht dabei so vor, daß man nach der Trennung der diastereomeren Ester jeweils diejenige Verbindung der Formel (III), die den (-)-Antipoden enthält, mit Base in Gegenwart eines Verdünnungsmittels behandelt. Die Reaktionsbedingungen entsprechen denjenigen, die im Falle des erfindungsgemäßen Verfahrens bei der Durchführung der zweiten Stufe in Frage kommen.

Der erfindungsgemäße Wirkstoff der Formel (I) besitzt sehr gute fungizide Eigenschaften und kann zur Bekämpfung unerwünschter Mikroorganismen praktisch eingesetzt werden.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit des Wirkstoffs in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Fungizid kann der erfindungsgemäße Wirkstoff mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gersten- bzw. des Getreidemehltaus (Erysiphe graminis). Weiterhin kann der erfindungsgemäße Wirkstoff besonders vorteilhaft als Getreidefungizid sowie

zur Bekämpfung von Pyricularia und Pellicularia an Reis verwendet werden.

Besonders hervorzuheben ist, daß der erfindungsgemäße Wirkstoff nicht nur eine protektive Wirkung entfaltet, sondern auch systemisch wirksam ist. So gelingt es, Pflanzen gegen Pilzbefall zu schützen, wenn man den Wirkstoff über den Boden und die Wurzel oder über das Saatgut den oberirdischen Teilen der Pflanze zuführt.

Der erfindungsgemäße Wirkstoff kann in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder

Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen oder organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Der erfindungsgemäße Wirkstoff kann in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Der Wirkstoff kann als solcher, in Form seiner Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Beim Einsatz des erfindungsgemäßen Wirkstoffes als Fungizid kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,01 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung des erfindungsgemäßen Wirkstoffes werden durch die nachfolgenden Beispiele veranschaulicht.

Herstellungsbeispiele:

Beispiel 1

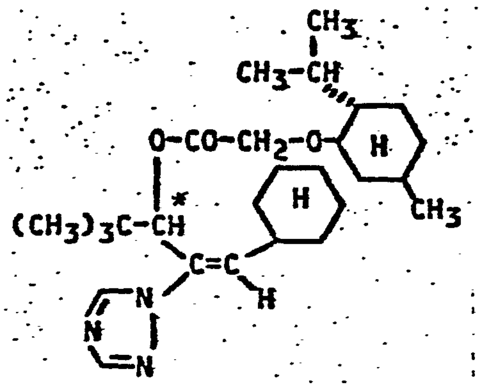

(III-1)

1. Stufe

12,0 g (0,0456 Mol) racemisches (E)-1-Cyclohexyl-4,4-dimethyl-3-hydroxy-2-(1,2,4-triazol-1-yl)-pent-1-en, 6,0 g (0,06 Mol) Triethylamin und 7,32 g (0,06 Mol) 4-Dimethylaminopyridin in 250 ml abolutem Tetrahydrofuran werden unter Rühren und Eiskühlung tropfenweise mit 11,7 g (0,0503 Mol) (-)-Menth-3-yloxyacetylchlorid versetzt und nach beendeter Zugabe weitere 20 Stunden bei 20°C bis 25°C gerührt. Zur Aufarbeitung gießt man das Reaktionsgemisch in 500 ml Wasser, extrahiert mehrfach mit Dichlormethan, trocknet die vereinigten organischen Extrakte über Natriumsulfat und entfernt das Lösungsmittel im Vakuum.

Man erhält 18,3 g (87,2 % der Theorie) an (E)-1-Cyclohexyl-4,4-dimethyl-3-(-)-(menth-3-yloxyacetyloxy)-2-(1,2,4-triazol-1-yl)-pent-1-en Diastereomeren-Gemisch als hochviskoses Öl.

Die Trennung in die diastereomeren Einzelkomponenten erfolgt mittels HPLC+) an einer Kieselgelsäule (Merck 5 - 20 µ Korngröße) mit dem Elutionsmittelgemisch Hexan, Tetrachlorkohlenstoff und Propionitril (7 : 2 : 1).

---

+) = High performance liquid chromatography (Hochauflösende Flüssigkeits-Chromatographie)

---

Als 1. Fraktion eluiert man 4,5 g (42,9 % der Theorie) an (-)-Menth-3-yloxyacetylester des (+)-Enantiomeren des (E)-1-Cyclohexyl-4,4-dimethyl-3-hydroxy-2-(1,2,4-triazol-1-yl)-pent-1-ens vom Brechungsindex $n_D^{20}$ = 1,4922. Als 2. Fraktion eluiert man 4,7 g (44,9 % der Theorie) an (-)-Menth-3-yloxyacetylester des (-)-Enantiomeren des (E)-1-Cyclohexyl-4,4-dimethyl-3-hydroxy-2-(1,2,4-triazol-1-yl)-pent-1-ens vom Brechungsindex $n_D^{20}$ = 1,4925.

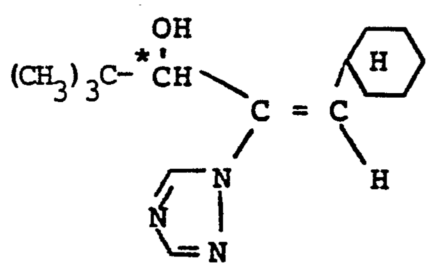

(+)-Antipode

2. Stufe

4,49 g (0,00978 Mol) desjenigen Diastereomeren, das aus der ersten Fraktion der im Beispiel 1, erste Stufe,

beschriebenen Eluierung gewonnen wurde, werden in 40 ml Methanol mit einer Lösung von 0,6 g (0,015 Mol) Natriumhydroxid in 5 ml Wasser versetzt und 3 Stunden bei 20°C bis 25°C gerührt. Zur Aufarbeitung verdünnt man mit 150 ml Wasser, extrahiert dreimal mit jeweils 120 ml Dichlormethan, wäscht die vereinigten organischen Extrakte mit Wasser, trocknet über Magnesiumsulfat und entfernt das Lösungsmittel im Vakuum. Der feste Rückstand wird mit Cyclohexan gewaschen und abgesaugt.

Man erhält 1,62 g (62,8 % der Theorie) des (+)-Antipoden des (E)-1-Cyclohexyl-4,4-dimethyl-3-hydroxy-2-(1,2,4-triazol-1-yl)-pent-1-ens vom Schmelzpunkt 159-161°C . Das Produkt besitzt eine optische Reinheit von 98%.

$[\alpha]_D^{20}$ = + 77,3° (c = 81,5 mg/10 ml $CHCl_3$)

Vergleichsbeispiel I

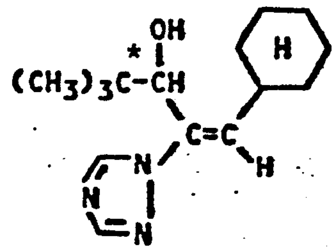

(-)-Antipode

4,65 g (0,0102 Mol) desjenigen Diastereomeren, das aus der zweiten Fraktion der im Beispiel 1, erste Stufe, beschriebenen Eluierung gewonnen wurde, werden in 40 ml Methanol mit einer Lösung von 0,6 g (0,015 Mol) Natriumhydroxid in 5 ml Wasser versetzt und 3 Stunden bei 20°C bis 25°C gerührt. Zur Aufarbeitung verdünnt man mit 150 ml Wasser, extrahiert dreimal mit jeweils 120 ml Dichlormethan, wäscht die vereinigten organischen Extrakte mit Wasser, trocknet über Magnesiumsulfat und entfernt das Lösungsmittel im Vakuum. Der feste Rückstand wird mit Cyclohexan gewaschen und abgesaugt.

Man erhält 1,9 g (70,9 % der Theorie) des (-)-Antipoden des (E)-1-Cyclohexyl-4,4-dimethyl-3-hydroxy-2-(1,2,4-triazol-1-yl)-pent-1-ens vom Schmelzpunkt 145°C bis 148°C.

Das Produkt besitzt eine optische Reinheit von 61 %.

$[\alpha]_D^{20}$ = - 48,6° (c = 87,5 mg/10 ml $CHCl_3$)

Vergleichsbeispiel II

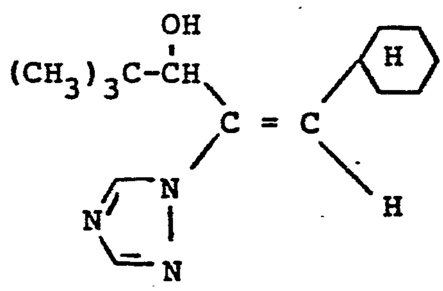

(Racemat)

26 g (0,1 Mol) (E)-1-Cyclohexyl-4,4-dimethyl-2-(1,2,4-triazol-1-yl)-pent-1-en-3-on werden in 200 ml Methanol aufgenommen und unter Rühren und Kühlen portionsweise mit 4,5 g Natriumborhydrid versetzt. Nach beendeter Reaktion wird das Reaktionsgemisch auf pH-6 eingestellt und eingeengt. Der Rückstand wird in 200 ml Methylenchlorid aufgenommen, mit gesättigter Natriumhydrogencarbonat-Lösung gewaschen, über Natriumsulfat getrocknet, filtriert und eingeengt. Der Rückstand wird aus Petrolether umkristallisiert. Man erhält 14,5 g (55 % der Theorie) racemisches (E)-1-Cyclohexyl-4,4-dimethyl-3-hydroxy-2-(1,2,4-triazol-1-yl)-pent-1-en vom Schmelzpunkt 131°C.

Herstellung des Ausgangsproduktes:

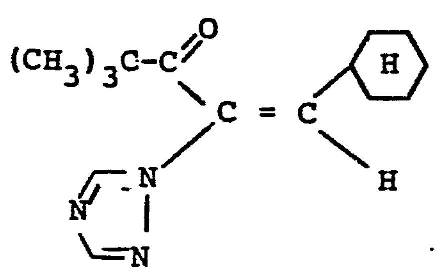

83,5 g (0,5 Mol) Pinakolyl-1,2,4-triazol, 60 g (0,54 Mol) Cyclohexanaldehyd, 4,2 g (0,05 Mol) Piperidin und 6 g (0,1 Mol) Eisessig in 300 ml Toluol werden am Wasserabscheider unter Rückfluß erhitzt, bis kein Wasser mehr übergeht. Nach dem Abkühlen der Reaktionslösung wird mit gesättigter Natriumchlorid-Lösung gewaschen, die organische Phase über Natriumsulfat getrocknet, filtriert und eingeengt. Der Rückstand wird in 500 ml Aceton aufgenommen und unter Rühren mit einer filtrierten Lösung von 90 g (0,25 Mol) Naphthalin-1,5-disulfonsäure in 500 ml Aceton versetzt.

Der zunächst ausfallende Niederschlag wird abgesaugt, das Filtrat weiter eingeengt und der erhaltene farblose kristalline Rückstand in 500 ml Methylenchlorid aufgenommen. Danach wird halbkonzentrierte wäßrige Natriumcarbonat-Lösung bis zur alkalischen Reaktion zugegeben. Die organische Phase wird abgetrennt, getrocknet, filtriert und eingeengt. Der ölige Rückstand wird in Petrolether aufgenommen, und der Kristallisation überlassen. Man erhält 64 g (49 % der Theorie) (E)-1-Cyclohexyl-4,4-dimethyl-2-(1,2,4-triazol-1-yl)-pent-1-en-3-on vom Schmelzpunkt 98 °C.

Beispiel A

Botrytis-Test (Bohne)/protektiv

Lösungsmittel: 4,7 Gewichtsteile Aceton
Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Wirkstoffe, Wirkstoffkonzentrationen und Versuchsergebnisse gehen aus der folgenden Tabelle hervor.

## Tabelle A

Botrytis-Test (Bohne) / protektiv

| Wirkstoff | Befall in % bei einer Wirkstoffkonzentration von 250 ppm |
|---|---|
| **bekannt** | |
| 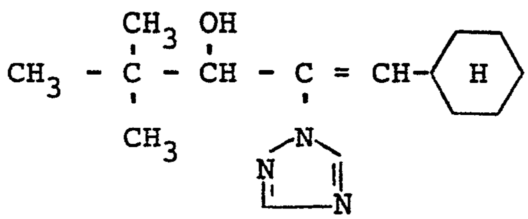 (Racemat) | 51 |
| **Erfindungsgemäß** | |
| 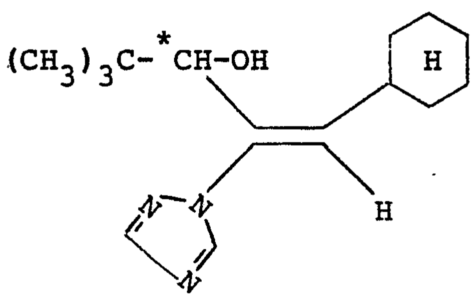 (+)-Antipode | 39 |

Beispiel B

Cochliobolus sativus-Test (Gerste) / protektiv

Lösungsmittel 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

Wirkstoffe, Wirkstoffkonzentrationen und Versuchsergebnisse gehen aus der nachfolgenden Tabelle hervor.

**T a b e l l e B**

Cochliobolus sativus-Test (Gerste)/protektiv

| Wirkstoff | Wirkstoffkonzentration in der Spritzbrühe in Gew.-% | Krankheitsbefall in % der unbehandelten Kontrolle |
|---|---|---|
| 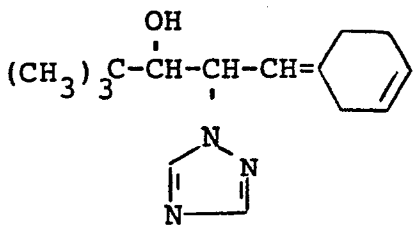<br>(bekannt) | 0,025 | 91,3 |
| 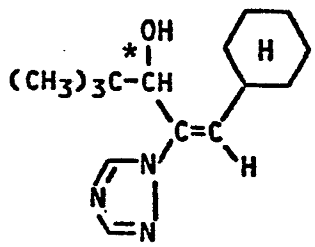<br>(-)-Antipode | 0,025 | 12,5 |
| 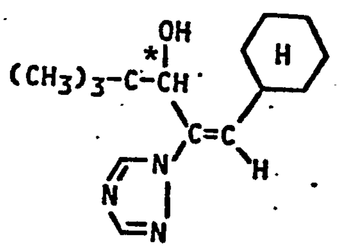<br>(+) -Antipode (erfindungsgemäß) | 0,025 | 0,0 |

Patentansprüche

1. (+)-Antipode des (E)-1-Cyclohexyl-4,4-Dimethyl-3-hydroxy-2-(1,2,4-triazol-1-yl)-pent-1-ens der Formel

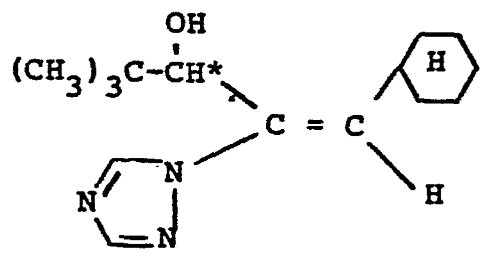

(I)

2. Verfahren zur Herstellung des (+)-Antipoden des (E)-1-Cyclohexyl-4,4-dimethyl-3-hydroxy-2-(1,2,4-triazol-1-yl)-pent-1-ens der Formel

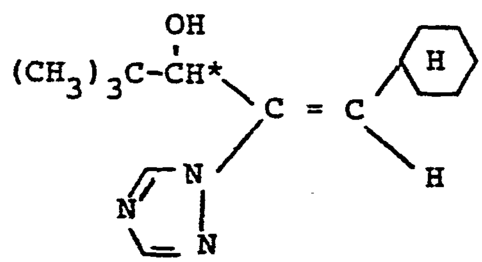

(I),

dadurch gekennzeichnet, daß man in einer ersten Stufe racemisches (E)-1-Cyclohexyl-4,4-dimethyl-3-hydroxy-2-(1,2,4-triazol-1-yl)-pent-1-en der Formel

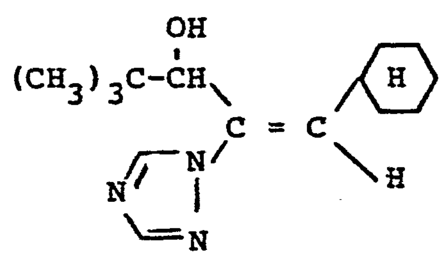

(Ia)

0114608

mit einem optisch aktiven Säurehalogenid der Formel

$$R - X - Hal \qquad (II)$$

in welcher

R für einen optisch aktiven Rest steht,

X für $-CO-$, $-SO_2-$ oder $-O-CH_2-CO-$steht und

Hal für Halogen steht,

in Gegenwart eines Verdünnungsmittels und gegebenenfalls in Gegenwart von Basen umsetzt, dann die so erhaltenen diastereomeren Ester der Formel

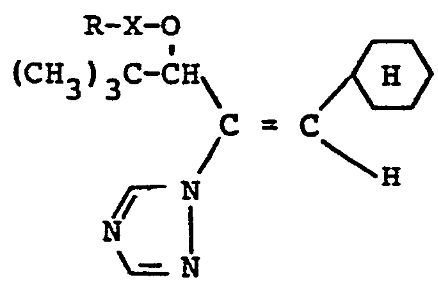

(III)

(Diastereomeren-Gemisch)

in welcher

R und X die oben angegebene Bedeutung haben,

aufgrund ihrer unterschiedlichen physikalischen Eigenschaften trennt,

und danach in einer zweiten Stufe den (+)-Antipoden des (E)-1-Cyclohexyl-4,4-dimethyl-3-hydroxy-2-(1,2,4-triazol-1-yl)-pent-1-ens aus dem entsprechenden Ester mit Hilfe von Basen in Gegenwart eines Verdünnungsmittels in Freiheit setzt.

3. Fungizide Mittel, gekennzeichnet durch einen Gehalt an dem (+)-Antipoden des (E)-1-Cyclohexyl-4,4-dimethyl-3-hydroxy-2-(2,3,4-triazol-1-yl)-pent-1-ens der Formel (I).

4. Verfahren zur Bekämpfung von Pilzen, dadurch gekennzeichnet, daß man den (+)-Antipoden des (E)-1-Cyclohexyl-4,4-dimethyl-3-hydroxy-2-(1,2,4-triazol-1-yl)-pent-1-ens der Formel (I) auf die Pilze und/oder deren Lebensraum ausbringt.

5. Verwendung des (+)-Antipoden des (E)-1-Cyclohexyl-4,4-dimethyl-3-hydroxy-2-(1,2,4-triazol-1-yl)-pent-1-ens der Formel (I) als Fungizid.

6. Verfahren zur Herstellung von fungiziden Mitteln, dadurch gekennzeichnet, daß man den (+)-Antipoden des (E)-1-Cyclohexyl-4,4-dimethyl-3-hydroxy-2-(1,2,4-triazol-1-yl)-pent-1-ens der Formel (I) mit Streckmitteln und/oder oberflächenaktiven Stoffen vermischt.